# EUROPEAN PATENT APPLICATION

(11) **EP 4 835 011 A1**
(43) Date of publication of application: **04.11.2026**
(21) Application number: 25888661.3
(22) Date of filing: 07.03.2025
(51) Int. Cl.: C09C 3/10, A61K 8/02, A61K 8/19, A61K 8/24, A61K 8/25, A61Q 1/02, C09C 1/00, G02B 5/20

(54) **INORGANIC NANOPARTICLE CORE-POLYMER SHELL STRUCTURAL COLORING MATERIAL, PARTICLE FILM, AND METHOD FOR PRODUCING NANOPARTICLE FILM**

(30) Priority: 07.11.2024 JP 2024195281
(71) Applicant: National University Corporation Kobe University, Kobe-shi, Hyogo 657-8501 (JP)
(72) Inventor: SUGIMOTO, Hiroshi, Kobe-shi, Hyogo 657-8501 (JP); FUJII, Minoru, Kobe-shi, Hyogo 657-8501 (JP)
(74) Representative: Casalonga
(86) International application number: PCT/JP2025/008640
(87) International publication number: WO 2026/100106

(57) **Abstract**

Providing polymer-shelled nanoparticles and a nanoparticle film which significantly suppress the formation of dimers and clusters by preventing direct contact between nanoparticle cores, thereby obtaining brighter color development in a target wavelength band. An inorganic nanoparticle core-polymer shell structural color material is used, consisting of an inorganic nanocrystal with a particle size of 40 to 300 nm and a refractive index of 3 or higher, surrounded by a polymer shell with a thickness of 20 to 200 nm. The polymer shell formed around the inorganic nanocrystals is based on hydroxyl groups present on the surface of the nanocrystals and surface-modified functional groups. For example, hydrophilic OH groups (hydroxyl groups) on the silicon surface are substituted with hydrophobic groups, and the silicon surface is modified and coated with N-isopropylacrylamide (NIPAM).

## Description

### TECHNICAL FIELD

The present invention relates to nanoparticles, and a nanoparticle film made of high refractive index materials such as silicon.

### BACKGROUND ART

Nanoparticles (100 to 200 nm) made of high refractive index materials such as silicon develop a color through Mie resonance, and are therefore expected to be used as structural color pigments and paints. The present inventors have proposed a dispersion solution of nanoparticles which does not change or fade in color and has high monochromaticity, and can achieve high resolution, as well as a method for producing the same (see PATENT LITERATURE 1). This approach focuses on the fact that silicon or the like exhibits significant light scattering in a visible wavelength range and can be used as a color-developing nanostructure without using a periodic array structure; and by forming a dispersion solution and controlling an average particle size, high scattered light is generated in the visible to near-infrared region with a single particle.

The present inventors have also proposed a nanoparticle film and a dispersion solution which can achieve high concealment with a small number of coating applications and allow for hue adjustment (see PATENT LITERATURE 2). This confirms that a monolayer, two-layer or three-layer particle film using nanoparticles (100 to 220 nm) of silicon or the like has high concealment, and achieves both high concealment and hue adjustment simultaneously by using nanoparticles with different particle size distributions.

However, there is a problem in a nanoparticle film (coating film) that color development changes when particles contact each other to form dimers or clusters. Therefore, a technology is required to prevent particles from contacting each other in a coating film.

On the other hand, PATENT LITERATURE 3 describes nano-semiconductor particles in which a silicon particle is a core portion and a surrounding shell portion is mainly composed of silicon oxide. In nano-semiconductor particles of PATENT LITERATURE 3, if the sum of the core portion particle size and the shell portion thickness exceed 100 nm, it exhibits bulk properties and a quantum size effect cannot be obtained, and therefore the particle size of the shelled particles is set at 100 nm or less. It also describes that the shell portion must have a thickness of 0.2 nm or more in order to isolate the cores from each other and avoid aggregation.

However, there is no description regarding suppressing the formation of dimers or clusters by forming a polymer shell on the surface of nanoparticles.

PATENT LITERATURE 4 describes particles having a shell of a silicon compound, the surface of which is modified with a silane coupling agent. The silica-coated nanoparticles described in PATENT LITERATURE 4 have high solvent dispersibility and are intended for immobilization on a substrate or the like, and are not intended for color development.

### [CITATION LIST]

### [PATENT LITERATURE]

[PATENT LITERATURE 1] JP 2021-025023 A
[PATENT LITERATURE 2] JP 2024-027742 A
[PATENT LITERATURE 3] WO2007/086321 A
[PATENT LITERATURE 4] JP 2010-001555 A

### SUMMARY

### [TECHNICAL PROBLEM]

As mentioned above, in a nanoparticle film (coating film), there was a problem that particles contact each other, and dimers or clusters are formed during drying or the like, causing a change in particle size and a corresponding change in color development due to Mie resonance.

In view of such circumstances, it is an object of the present invention to provide polymer-shelled nanoparticles and a nanoparticle film which significantly suppress the formation of dimers and clusters by preventing direct contact between nanoparticle cores, thereby obtaining brighter color development in a target wavelength band.

### [SOLUTION TO PROBLEM]

In order to solve the above problems, the polymer-shelled nanoparticle of the present invention is an inorganic nanoparticle core-polymer shell structural color material including an inorganic nanocrystal with a particle size of 40 to 300 nm and a refractive index of 3 or higher, surrounded by a polymer shell with a thickness of 20 to 200 nm.

A polymer shell having a predetermined thickness is formed on the surface of nanoparticles made of high refractive index materials, such as silicon which develops a color through Mie resonance, thereby preventing nanoparticle cores from directly contacting each other in a nanoparticle film (coating film), and suppressing the formation of dimers and clusters to develop a bright color.

According to the above configuration, the coating film of structural color particles can be expected to enhance brightness and chroma, thereby broadening its applications as a paint. Polymer-shelled nanoparticles may promote chemical bonding with a binder, thus enabling the formation of a more stable coating film.

Here, Mie resonance refers to a phenomenon where, when light of wavelength λ (nm) is incident on inorganic nanoparticles (refractive index n), the effective wavelength in the nanoparticles becomes λ/n (nm), and when this effective wavelength λ/n (nm) of light becomes equal to the diameter of the nanoparticles, a standing wave is formed, and the lowest-order Mie resonance occurs. The particle size of the nanoparticles means an average particle size, and a certain type of particle size distribution is in a range (for example, 4 to 50%) obtained by dividing the standard deviation by the average particle size.

In the polymer-shelled nanoparticles of the present invention, the particle size is preferably 100 to 300 nm, and more preferably 100 to 220 nm. By selecting the particle size of 100 to 220 nm, when the color development through Mie resonance is visible light, the particle size is restricted within a range of 100 to 220 nm.

Examples of the inorganic nanocrystal with a refractive index of 3 or higher include silicon (Si), GaAs, GaP, InP and the like. The refractive index of silicon is 4.32, the refractive index of GaAs which is an inorganic compound is 4.27, the refractive index of GaP is 3.6, and the refractive index of InP is 3.0. Note that the refractive index depends on wavelength, but it refers to the value at a wavelength of 500 nm.

The inorganic nanocrystal preferably has a refractive index of 4 or more, such as silicon or GaAs. More preferably, it is silicon, which is the second most abundant element on Earth after oxygen, found in soil, rocks, natural water, trees, plants and the like, and is widely used in semiconductors.

In the inorganic nanoparticle core-polymer shell structural color material, the polymer shell formed around the inorganic nanocrystal is preferably any one of polystyrene, polyacrylic, polyethylene glycol, and N-isopropylacrylamide (NIPAM). Some polymer materials have both hydrophobic and hydrophilic groups, while other are either hydrophobic or hydrophilic.

Preferably, the polymer shell formed around the inorganic nanocrystal is formed by bonding or adsorbing to a hydroxyl group or a surface-modified functional group on the surface of the inorganic nanocrystal. For example, hydrophilic OH groups (hydroxyl groups) on the silicon surface are substituted with hydrophobic groups, and the silicon surface is modified and coated with N-isopropylacrylamide (NIPAM).

The inorganic nanoparticle core-polymer shell structural color material may be configured such that the inorganic nanocrystal, which is an inorganic nanoparticle core, has a particle size of 100 to 200 nm and develops a structural color in the visible light region. Here, when the particle size is 100 to 110 nm, a pinkish structural color is developed, when the particle size is 115 to 125 nm, a bluish structural color is developed, when the particle size is 130 to 140 nm, a greenish structural color is developed, when the particle size is 145 to 160 nm, a yellowish structural color is developed; and when the particle size is 165 to 200 nm, it is possible to prepare a flesh-colored structural color material.

The inorganic nanoparticle core-polymer shell structural color material may be configured such that particles having particle size distribution peaks at approximately 100 nm, approximately 130 nm and approximately 200 nm, respectively, coexist in the inorganic nanocrystal to develop a white structural color. The white color is developed by mixing three types of particles: blue color development (particle size distribution with a peak around 100 nm), green color development (particle size distribution with a peak around 130 nm) and red color development (particle size distribution with a peak around 200 nm). The particle size distribution here refers to the distribution with a width of approximately ±10%.

The inorganic nanoparticle core-polymer shell structural color material may also be configured such that the inorganic nanocrystal has a particle size of 200 to 300 nm and develops a structural color in the near-infrared region. The particle size is unified to the size which develops a structural color in the near-infrared region.

The inorganic nanoparticle core-polymer shell structural color material may be configured such that the inorganic nanocrystal has a particle size of 40 to 99 nm and develops a structural color in the ultraviolet region. The particle size is unified to the size which develops a structural color in the ultraviolet region.

The inorganic nanocrystal is preferably selected from Si, GaAs, GaP and InP. These are specific inorganic materials with a refractive index of 3 or higher.

The nanoparticle film of the present invention is a monolayer film, a multilayer film or a laminated film formed from the above inorganic nanoparticle core-polymer shell structural color material, and a surface packing ratio of the nanoparticles in a monolayer is 15 to 74%.

Using this nanoparticle film, cosmetic products and highly concealing nanoparticle films can be produced. Examples of cosmetic products include foundation, lipstick, eyeshadow and the like.

The method for producing a nanoparticle film of the present invention is a method for producing a particle film comprising the above inorganic nanoparticle core-polymer shell structural color material, the method including steps of: 1) preparing inorganic nanoparticles with a refractive index of 3 or higher to have a particle size of 40 to 300 nm; 2) coating the inorganic nanoparticles with a polymer shell with a thickness of 20 to 200 nm; and 3) floating the inorganic nanoparticles coated with the polymer shell on a liquid surface and transferring them to a substrate.

In the above method for producing a nanoparticle film, it is preferable to further include a step of washing the particle film on the substrate with an organic solvent or performing an oxygen plasma treatment to remove only the polymer shell.

### [ADVANTAGEOUS EFFECTS OF INVENTION]

According to the polymer-shelled nanoparticles and the nanoparticle film of the present invention, the formation of dimers and clusters can be significantly suppressed by preventing nanoparticle cores from directly contacting each other, and the effect of obtaining brighter color development in a target wavelength band is exerted.

### BRIEF DESCRIPTION OF DRAWINGS

FIG. 1 is a schematic diagram regarding the dispersion of Si nanoparticles.
FIG. 2 is a schematic diagram in which a polymer shell is formed using functional groups on the surface of Si nanoparticles.
FIG. 3 is a flowchart for producing a Si nanoparticle film.
FIG. 4 is an optical microscope image of a Si nanoparticle film after particle size separation.
FIG. 5 is an enlarged view of the optical microscope image of a Si nanoparticle film after particle size separation.
FIG. 6 is an explanatory diagram in which a polymer shell is formed on the surface of Si nanoparticles (Example 1).
FIG. 7 is a schematic diagram of a method for producing a Si nanoparticle film.
FIG. 8 is an SEM image of a Si nanoparticle film (after oxygen plasma treatment).
FIG. 9 is a dark-field image diagram of a Si nanoparticle film.
FIG. 10 is an extinction spectrum diagram of a Si nanoparticle dispersion.
FIG. 11 is a reflection spectrum diagram of a Si nanoparticle film (after oxygen plasma treatment).
FIG. 12 is a color space obtained from the reflection spectrum of a Si nanoparticle film (difference with and without a surface shell).

### DESCRIPTION OF EMBODIMENTS

Hereinafter, an example of the embodiments of the present invention will be described in detail with reference to the drawings. The scope of the present invention is not limited to the following examples and illustrated examples, and various changes and modifications are possible.

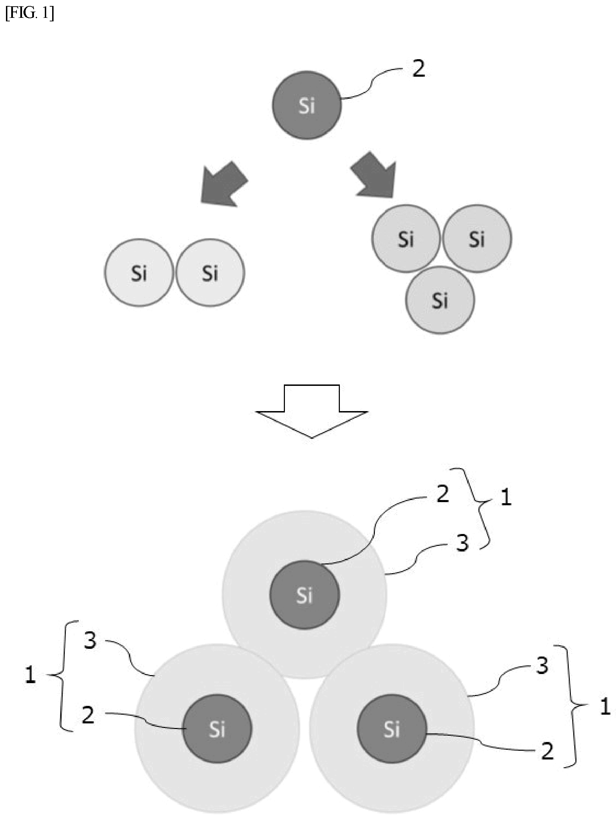

FIG. 1 is a schematic diagram regarding the dispersion of Si nanoparticles, and current problems will be described using FIG. 1. As shown in FIG. 1, isolated Si nanoparticles 2, which are inorganic nanocrystals, develop a color through Mie resonance. However, when dimers or clusters (multimers) are formed, the color development changes, thus causing a decrease in chroma and brightness. Therefore, by forming a polymer shell with a predetermined thickness (thickness of 20 to 200 nm) around the Si nanoparticles 2, the nanoparticles are prevented from contacting each other to give an inorganic nanoparticle core-polymer shell structural color material. As used herein, the nanoparticles refer to nanometer-sized particles, Si nanoparticles are spherical particles formed from crystalline Si having a particle size (diameter) in the nanometer size, and Si is a nanoparticle with a positive real part of its dielectric constant and exhibits Mie resonance.

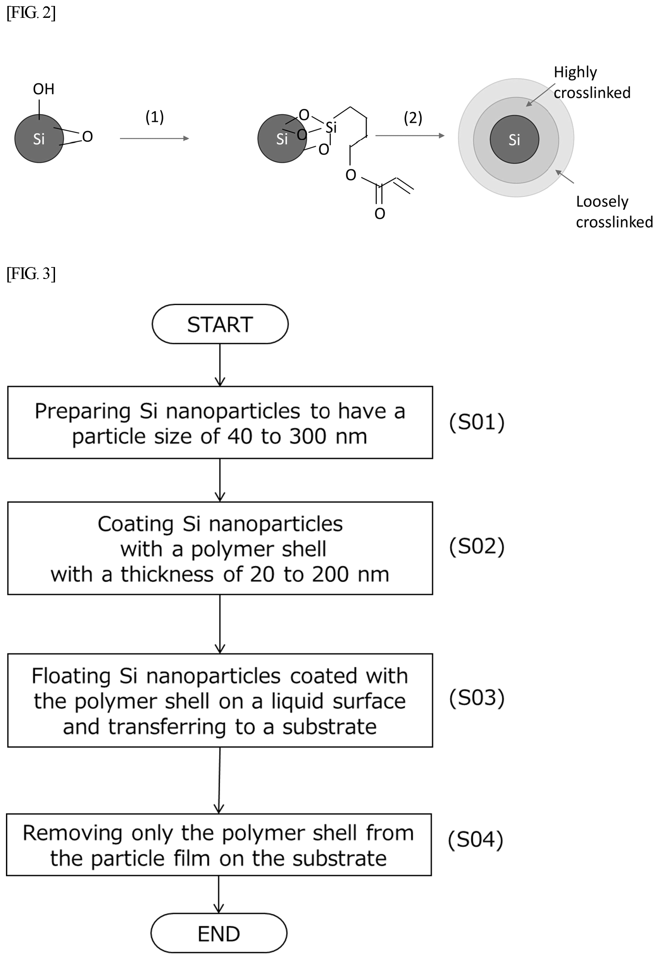

FIG. 2 shows a schematic diagram in which a polymer shell is formed using functional groups on the surface of Si nanoparticles.

First, Si nanoparticles are dispersed in a solvent. A solution containing functional groups to be modified on the surface of the Si nanoparticles is added to modify the functional groups on the surface of the Si nanoparticles (see FIG. 2(1)).

A polymer is bonded or adsorbed to the surface-modified functional groups to form a polymer shell (see FIG. 2(2)). The functional groups modified on the surface of the Si nanoparticles are tightly crosslinked, while the polymer bonded or adsorbed to the functional groups is relatively loosely crosslinked.

FIG. 3 shows a flowchart for producing a Si nanoparticle film. First, Si nanoparticles are prepared to have a particle size of 40 to 300 nm (Step S01). For example, using commercially available silicon monoxide powder as a raw material, annealing is performed under temperature conditions within a range from below a melting point of single-crystal silicon (1,414°C) to above the melting point (1,350 to 1,700°C), for example, at 1,450°C for 30 minutes in a nitrogen (N₂) atmosphere. By controlling the annealing temperature conditions, the particle size of crystalline Si nanoparticles can be controlled. The powder obtained after the annealing process consists of silicon dioxide particles containing Si nanoparticles. The annealed powder is etched using hydrofluoric acid (HF). By etching with hydrofluoric acid, only the silicon dioxide portion is etched, and the Si nanoparticles contained within the particles are extracted. After etching, the etching solution is replaced with alcohol using methanol as a polar solvent. Thereafter, the mixture is stirred with an ultrasonic homogenizer to disperse the Si nanoparticles in the alcohol, and the particle size of the Si nanoparticles in the dispersion is unified using a density gradient centrifugation method (2,000 to 3,000 G; 60 minutes). Through the above processes, a dispersion of Si nanoparticles having a controlled particle size is produced.

It is possible to use as, other methods for unifying the particle size in the dispersion of nanoparticles, the density gradient centrifugation method and a particle size selection method by adding a poor solvent. When using the density gradient centrifugation method, it is preferable to perform centrifugation with high particle size resolution by allowing the centrifuge tubes to lie on a horizontal plane. By allowing the tubes to lie on a horizontal plane and performing centrifugation in a lateral direction, the direction of centrifugal force becomes identical to the longitudinal direction toward the bottom of the centrifuge tube, leading to an increase in sedimentation path length, thus making it possible to improve the resolution of the density gradient centrifugation.

Next, the Si nanoparticles are coated with a polymer shell with a thickness of 20 to 200 nm (Step S02). Then, the Si nanoparticles coated with the polymer shell are floated on a liquid surface and transferred to a substrate (Step S03). For example, a film is formed by self-assembling the Si nanoparticles coated with the polymer shell on a glass substrate. Alternatively, a film may be formed by spin-coating or drop-coating a dispersion of the Si nanoparticles coated with the polymer shell onto a substrate.

Thereafter, only the polymer shell is removed from the particle film on the substrate (Step S04).

A comparison in particle size distribution between Si nanoparticles and polymer shell-coated Si nanoparticles will be described. The particle size in a state dispersed in a solution was measured using dynamic light scattering (DLS). The average particle sizes of three types of uncoated Si nanoparticles and two types of polymer shell-coated Si nanoparticles (NIPAM: 50 mM, 25 mM) are 175 nm, 275 nm and 399 nm, respectively, leading to an increase in particle size increased due to the polymer shell coating. Therefore, the shell thickness is determined to be 224 divided by 2 = 112 nm when NIPAM is 50 mM, and 100 divided by 2 = 50 nm when the shell thickness is 25 mM.

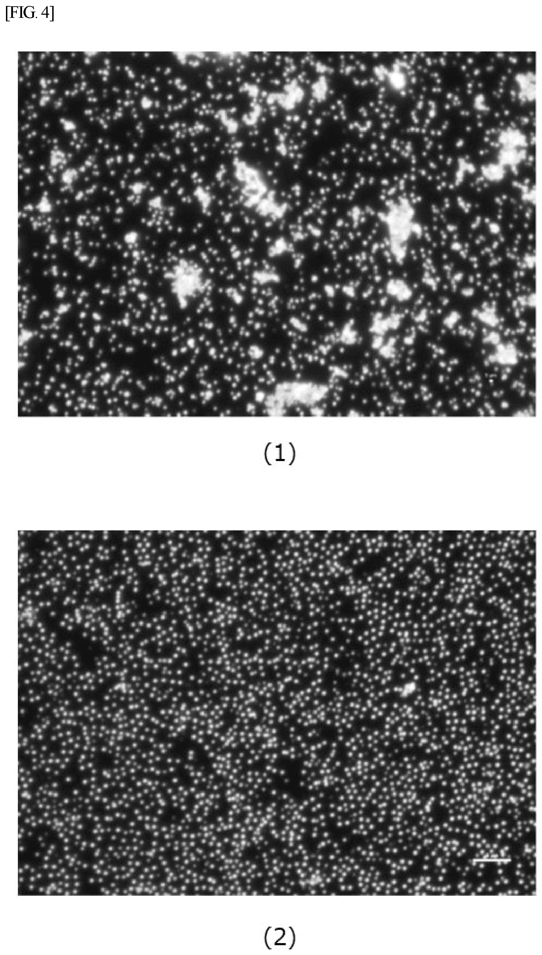

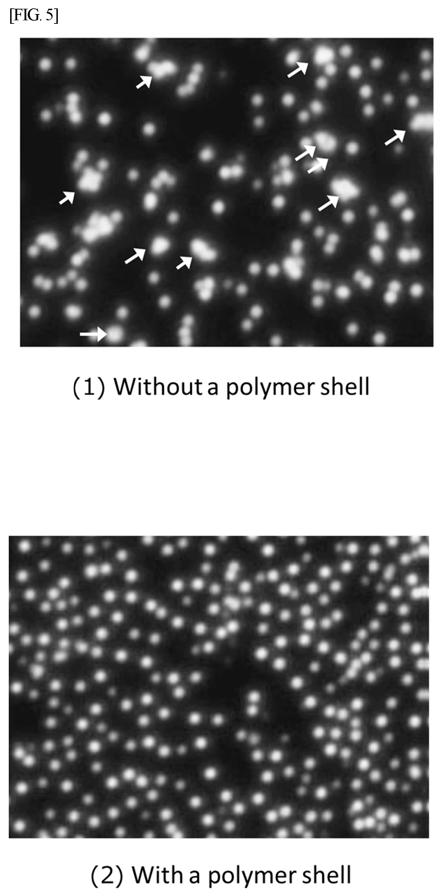

FIG. 4 is an optical microscope image of the produced Si nanoparticle film after particle size separation, and FIG. 5 shows an enlarged view thereof. In FIG. 4 and FIG. 5, (1) shows the case without a polymer shell, and (2) shows the case with a polymer shell. As shown in FIG. 5(1), in the case without a polymer shell, it can be seen that many clusters exist as indicated by the arrows in the image, whereas in the case with a polymer shell as shown in FIG. 5(2), individual particles can be clearly observed.

### [Example 1]

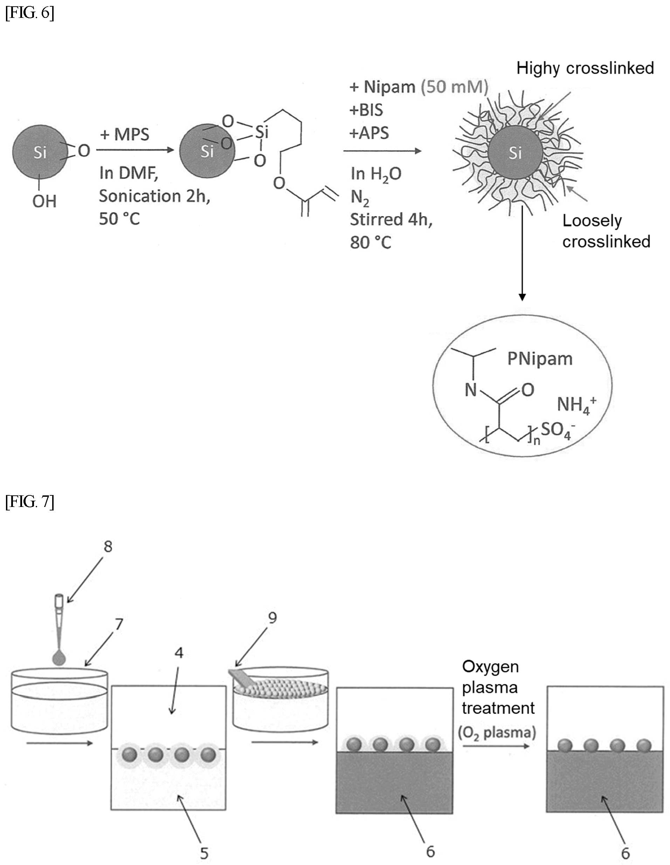

An example of an embodiment for forming a polymer shell on the surface of Si nanoparticles will be described. FIG. 6 shows an explanatory diagram in which a polymer shell is formed on the surface of Si nanoparticles, and FIG. 7 shows a schematic diagram of a method for producing a Si nanoparticle film.

First, a dispersion in which Si nanoparticles are dispersed in alcohol is centrifuged to remove the alcohol and dried overnight at 70°C. Thereafter, the Si nanoparticles are dispersed in DMF (N,N-dimethylformamide) (concentration of 0.04% by weight). This mixture is subjected to an ultrasonic treatment, and MPS ((trimethoxysilyl)propyl methacrylate) is added (100 or more molecules per 1 nm² of the surface area of the Si nanoparticles), followed by reaction and further ultrasonic treatment for 2 hours at 50°C. Thereafter, washing with alcohol is performed 4 to 5 times.

Next, 25 mM or 50 mM of NIPAM, BIS as a crosslinking agent, and ammonium persulfate (APS) as a polymerization accelerator are added to a nitrogen-purged glass container. While heating to 80°C and stirring, the dispersion of MPS-modified Si nanoparticles is added little by little using a syringe to form a polymer (poly(N-isopropylacrylamide)) shell on the surface of the Si nanoparticles.

The production of the Si nanoparticle film in the laboratory was performed as shown in FIG. 7. The polymer shell-coated Si nanoparticles were dispersed in alcohol, and the dispersion was gently dropped onto a water surface to float the particles, which were then transferred to a substrate to form a film.

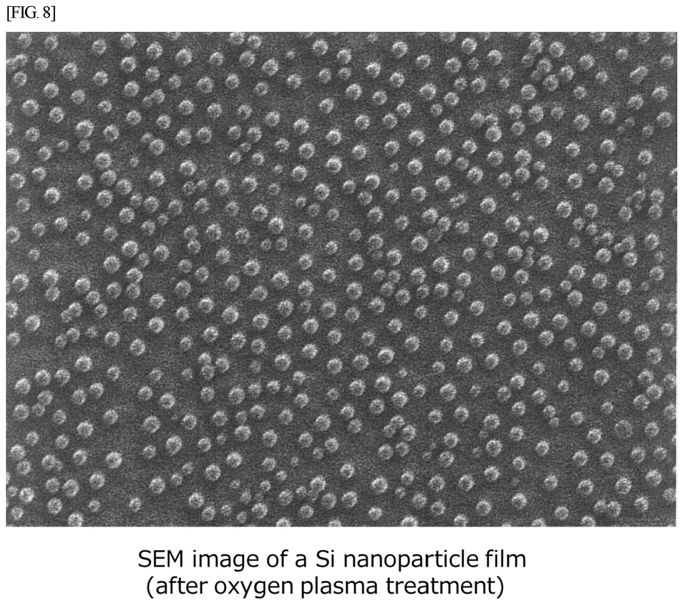

FIG. 8 shows an SEM image of the Si nanoparticle film after an oxygen plasma treatment. The Si nanoparticles have an average particle size (Dₐᵥₑ) of 188.31 nm. As shown in FIG. 8, it can be seen that the crystalline Si nanoparticles constituting the produced Si nanoparticle film have a circularity close to that of a true sphere.

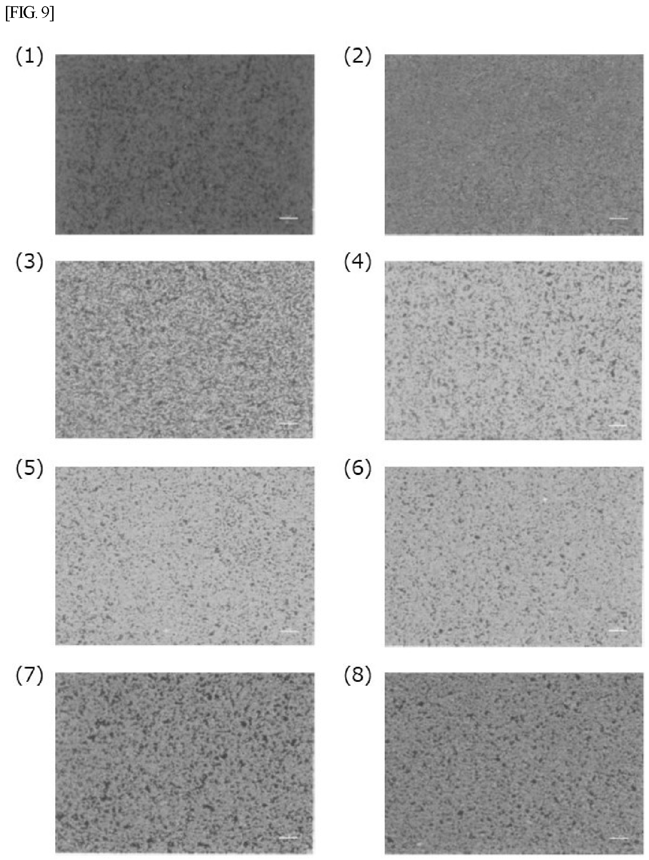

FIG. 9 shows a dark-field image diagram of the Si nanoparticle film.

FIG. 9(1) to (8) are dark-field images of the Si nanoparticle films of samples (F1 to F8), respectively, in which the average particle size (Dₐᵥₑ) and the standard deviation divided by the average particle diameter (CV) of the Si nanoparticles are as shown in Table 1 below. The differences in hue were confirmed by controlling the average particle diameter within a range of approximately 110 to 200 nm ± 10%.

**[Table 1]**

| Sample | F1 | F2 | F3 | F4 | F5 | F6 | F7 | F8 |
|---|---|---|---|---|---|---|---|---|
| Dₐᵥₑ | 109.52 | 133.46 | 151.56 | 165.13 | 176.89 | 188.31 | 196.33 | 207.89 |
| CV | 13.4 | 9.8 | 8.7 | 8.2 | 8.3 | 7.7 | 8.6 | 7.3 |

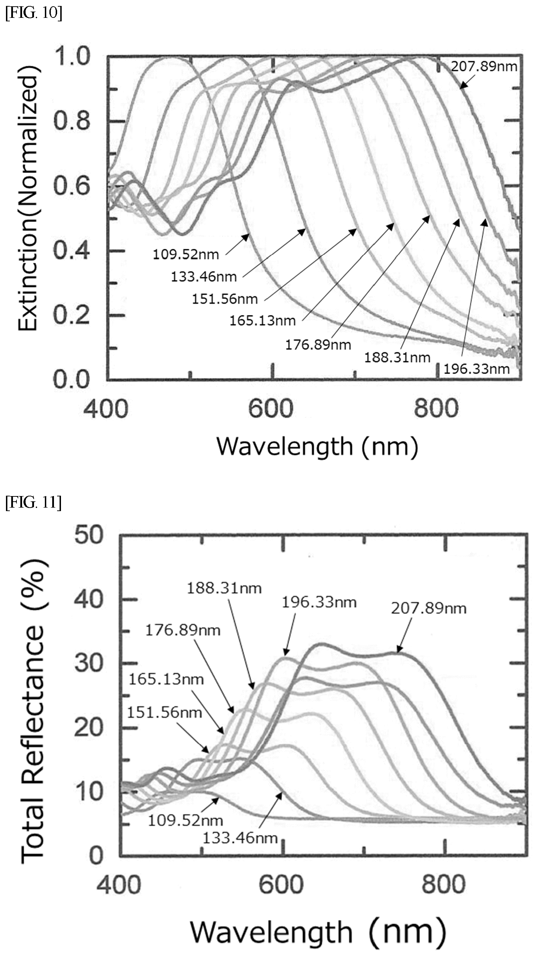

FIG. 10 shows an extinction spectrum of a Si nanoparticle dispersion. It can be seen that as the average particle size increases, the spectral peaks shift toward longer wavelengths.

FIG. 11 shows the total reflection spectra of the Si nanoparticle films (after oxygen plasma treatment). From FIG. 11, reflecting the extinction spectra of the dispersions in FIG. 10, it can be seen that the reflectance spectra change within a range of approximately 450 to 800 nm as the nanoparticle size increases. The total reflectance is expressed as the ratio of the sum of specular reflected light and diffusely reflected light to the incident light.

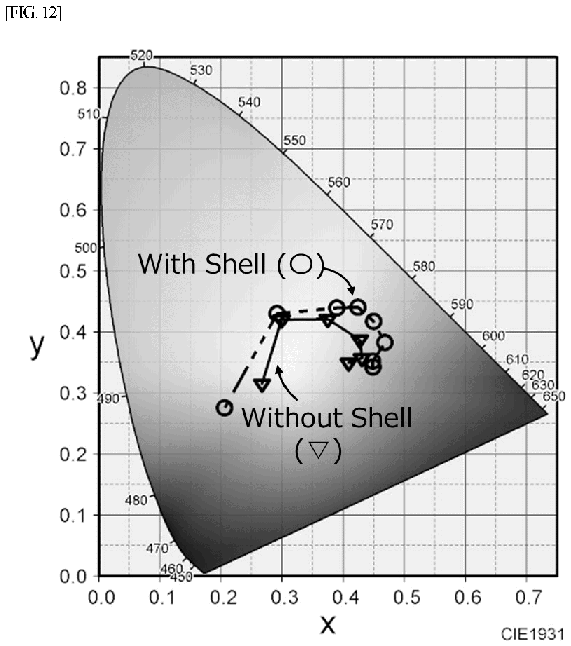

FIG. 12 shows a color space obtained from the reflection spectra of Si nanoparticle films with and without a surface shell. FIG. 12 shows the CIE 1931 color space. The CIE 1931 color space quantitatively represents the relationship between visible light and color in human color vision. From FIG. 12, those having a polymer shell formed on the Si nanoparticle surface exhibit variations over a wider range than the Si nanoparticle surface without a polymer shell. This reveals that the color gamut is expanded due to the suppression of cluster formation by the polymer shell.

### INDUSTRIAL APPLICABILITY

The present invention is useful for cosmetics, particularly foundations which are effective in small amounts, and highly concealing inks.

### REFERENCE SIGNS LIST

1: Structural color material
2: Si nanoparticles
3: Polymer shell
4: Air
5: Water
6: Glass substrate
7: Beaker
8: Dropper
9: Glass rod

## Claims

1. An inorganic nanoparticle core-polymer shell structural color material comprising an inorganic nanocrystal with a particle size of 40 to 300 nm and a refractive index of 3 or higher, surrounded by a polymer shell with a thickness of 20 to 200 nm.

2. The inorganic nanoparticle core-polymer shell structural color material according to claim 1, wherein the polymer shell is any one of polystyrene, polyacrylic, polyethylene glycol and N-isopropylacrylamide (NIPAM).

3. The inorganic nanoparticle core-polymer shell structural color material according to claim 1, wherein the polymer shell is formed by bonding or adsorbing to a hydroxyl group or a surface-modified functional group on the surface of the inorganic nanocrystal.

4. The inorganic nanoparticle core-polymer shell structural color material according to claim 1, wherein the inorganic nanocrystal has a particle size of 100 to 200 nm and develops a structural color in the visible light region.

5. The inorganic nanoparticle core-polymer shell structural color material according to claim 1, wherein particles having particle size distribution peaks at approximately 100 nm, approximately 130 nm and approximately 200 nm, respectively, coexist in the inorganic nanocrystal, and develop a white structural color.

6. The inorganic nanoparticle core-polymer shell structural color material according to claim 1, wherein the inorganic nanocrystal has a particle size of 200 to 300 nm and develops a structural color in the near-infrared region.

7. The inorganic nanoparticle core-polymer shell structural color material according to claim 1, wherein the inorganic nanocrystal has a particle size of 40 to 99 nm and develops a structural color in the ultraviolet region.

8. The inorganic nanoparticle core-polymer shell structural color material according to claim 1, wherein the inorganic nanocrystal is selected from Si, GaAs, GaP and InP.

9. A particle film comprising an inorganic nanoparticle core-polymer shell structure color material, which is a monolayer film, a multilayer film or a laminated film formed from the inorganic nanoparticle core-polymer shell structure color material according to any one of claims 1 to 8, wherein a surface packing ratio of nanoparticles in a monolayer is 15 to 74%.

10. A cosmetic product using a particle film comprising the inorganic nanoparticle core-polymer shell structural color material according to claim 9.

11. A highly concealing nanoparticle film having the particle film comprising the inorganic nanoparticle core-polymer shell structural color material according to claim 9.

12. A method for producing a particle film comprising the inorganic nanoparticle core-polymer shell structural color material according to any one of claims 1 to 8, the method comprising steps of:
preparing inorganic nanoparticles with a refractive index of 3 or higher to have a particle size of 40 to 300 nm;
coating the inorganic nanoparticles with a polymer shell with a thickness of 20 to 200 nm; and
floating the inorganic nanoparticles coated with the polymer shell on a liquid surface and transferring them to a substrate.

13. The method for producing a nanoparticle film according to claim 12, further comprising a step of washing the particle film on the substrate with an organic solvent or performing an oxygen plasma treatment to remove only the polymer shell.
